# EUROPEAN PATENT SPECIFICATION

(11) **EP 1 878 731 B1**
(45) Date of publication and mention of the grant of the patent: **20.08.2008**
(21) Application number: 06013150.5
(22) Date of filing: 26.06.2006
(51) Int. Cl.: C07D 277/82

(54) **Process for producing pramipexole**
Verfahren zur Herstellung von Pramipexole
Procédé pour la préparation du Pramipexole

(43) Date of publication of application: 16.01.2008
(73) Proprietor: Helm AG, 20097 Hamburg (DE); CF Pharma Gyogyszergyarto Kft., 1097 Budapest (HU)
(72) Inventor: Gegö, Csaba Lehel, 1181 Budapest (HU); Fejes, Imre, 1012 Budapest (HU); Garaczi, Sándor, 1048 Budapest (HU); Kovács, Imre, 4034 Decevren (HU); Lukács, Ferenc, 2134 Kistarcsa (HU); Majercsik, Orsolya, 1161 Budapest (HU); Schneider, Geza, 1097 Budapest (HU)
(74) Representative: Teipel, Stephan

(56) References cited:
- WO-A-02/22591
- WO-A-97/03076
- WO-A-20/06040279
- WO-A2-20/05092871
- WO-A2-20/06012277
- US-A1- 2003 153 568
- SCHNEIDER C S ET AL: "Dopamine autoreceptor agonists: resolution and pharmacological activity of 2,6-diaminotetrahydrobenzothiazole and aminothiazole analogue of apomorphine" JOURNAL OF MEDICINAL CHEMISTRY, AMERICAN CHEMICAL SOCIETY. WASHINGTON, US, vol. 30, no. 3, March 1987 (1987-03), pages 494-498, XP002186199 ISSN: 0022-2623

## Description

The present invention relates to a process for preparing (S)-pramipexole and to certain intermediates useful therein.

(S)-Pramipexole [(*S*)-4,5,6,7-tetrahydro*-N*⁶-*n*-propyl-2,6-benzothiazolediamine], of formula I (the asterisk * indicates the chiral carbon atom) 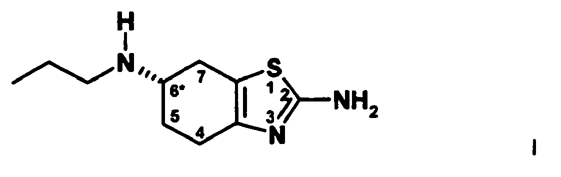 is a dopamine-D3/D2 agonist, the synthesis of which is described in EP 186 087 A1 and its counterpart, US 4,886,812 A1. It is suitable for the treatment of central nervous, neuropsychiatric diseases, particularly schizophrenia, for the treatment of Parkinsonism and Parkinson's disease and/or for treating circulatory disorders, particularly hypertension. US patent No. 6,001,861 describes the use of pramipexole for the treatment of restless legs syndrome.

International patent application WO 01/62249 A1 describes the treatment of addictive disorders, psychoactive substance use disorders, intoxication disorders, inhalation disorders, alcohol addiction, tobacco addiction and/or nicotine addiction resulting in smoking cessation, involving administration of a therapeutically effective, nontoxic dose of pramipexole.

Further, DE 101 37 633 A1 discloses that pramipexole can be used for the prevention and/or treatment of attention deficit hyperactivity disorder (ADHD). US 2004/0266794 A1 suggests pramipexole for the reduction of excessive food intake for children.

Pramipexole contains a chiral carbon atom and is commercially sold as a dihydrochloride (monohydrate) salt of the (*S*)-enantiomer thereof. The dopaminergic activity of the (*S*)-isomer is twice as high as that of the (*R*)-enantiomer.

There are several processes known in the art for preparing pramipexole, which can be classified into three general groups.

One general method for preparing compounds of the formula (C), applicable to a synthesis of pramipexole, uses substituted amino ketones (A) as key intermediates. (A) is halogenated to give α-halogeno-ketone (B) which is treated with thiourea to form 2,6-diamino-tetrahydrobenzothiazole derivatives (C)

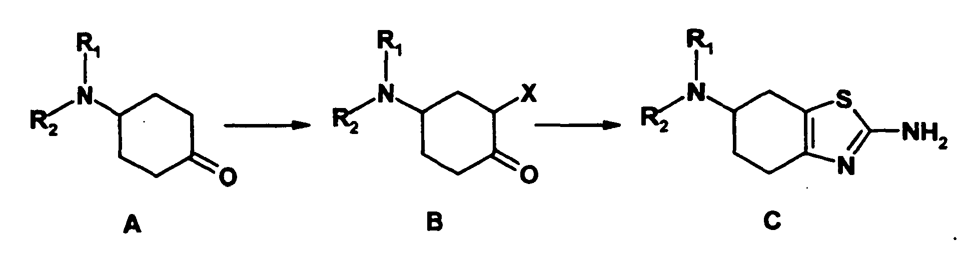

In EP 186 087 A1, EP 207 696 A1 and by Schneider and Mierau in J. Med. Chem. 30, 494 (1987) the application of appropriately protected amino-cyclohexanone derivatives (A) is suggested. To form the N-propyl moiety of pramipexole three different strategies, outlined below, have been proposed:
(i) In compound A, R₁ is an n-propyl group, R₂ is a protecting group; the protecting group is removed after conversion to compound C.
(ii) In compound A, R₁ is a propionyl group, R₂ is hydrogen; the reduction of the propionyl group to obtain an n-propyl group is done after conversion to compound C.
(iii) In compound A, R₁ is hydrogen or a protecting group and R₂ is a protecting group; the deprotection, propionylation and reduction to obtain an n-propyl group is done after conversion to compound C.

Alternatively, in WO 2004/026850 A1 a process is disclosed which allows the preparation of compound C (R₁ is n-propyl, R₂ is hydrogen) from the ketone A (R₁ is n-propyl, R₂ is hydrogen), without the requirement of a protective group at the nitrogen atom of the amino substituent of ketone A.

A second approach, disclosed in US 2004/0029936 A1, to obtain pramipexole and related products involves the masked bromo-1,4-cyclohexanedione D (R represents an alkoxy group) as key intermediate. D can be obtained by selective monobromination of 1,4-cyclohexanedione in an alcoholic solvent. Condensation with thiourea gives 6-oxo-tetrahydrobenzothiazole of formula E. In the last step E is subjected to reductive amination with the corresponding alkyl amine.

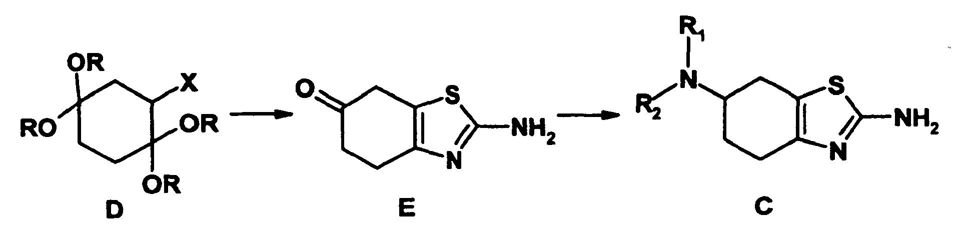

The third approach towards the synthesis of pramipexole is disclosed in WO 2005/092871 A2. The amino function is introduced by application of the Schmidt, Hofmann, Lossen and Curtius reactions in position 6 of the tetrahydrobenzothiazole ring (R₁ and R₂ are hydrogen). The appropriate precursors of these rearrangements can be prepared or generated in situ from the 6-carboxylic acid derivative of formula G (X is a hydroxy group). The latter compound was prepared in a three-step procedure involving bromination of 4-oxo-cyclohexanecarboxylic acid esters of formula F, cyclisation with thiourea and hydrolysis of the ester function. The propylation of C (R₁ and R₂ are hydrogen) was carried out as described above at item (iii).

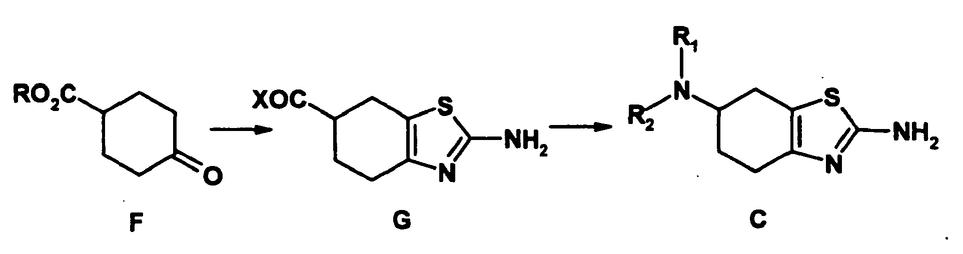

The first example of a process for preparing optically pure pramipexole was disclosed by Schneider and Mierau in J. Med. Chem. 30, 494 (1987). The optical resolution of 2,6-diamino-4,5,6,7-tetrahydrobenzothiazole (formula C, R₁ and R₂ are hydrogen) was accomplished with L-(+)-tartaric acid. The L-tartrate salt was converted into the corresponding base, which on reaction with propionic anhydrate afforded the enantiomeric propionamido compound. Reduction with diborane gave (*S*)-pramipexole (I).

The resolution of racemic pramipexole using many of the conventional optically active acids, as proposed in EP 186 087 A1, reveals that the degree of enrichment of the desired enantiomer was low (compare US 2002/0103240 A1). The latter patent application discloses inter alia a process for resolving or enriching (R,S)-pramipexole into optical isomers, using monovalent salts as substrate. The monovalent salts are reacted with an optically active acid to form diastereomeric mixed salts, which in turn facilitates resolution of the (R)- and (*S*)-enantiomers via preferential precipitation.

A stereoselective process for the preparation of pramipexole enriched in the desired enantiomer is disclosed in WO 02/22590 A1 and WO 02/22591 A1. The process utilizes an enantioselective reductive amination. However, the degree of enantioselectivity is not satisfactory.

US 2004/0029936 A1 discloses a process to the asymmetric synthesis of (S)-pramipexole, which uses a chiral amine, convertible to propylamine as reagent in the reductive amination step with compound E. The efficiency of chiral induction proved to be unsatisfactory.

WO 2005/092871 A2 describes a process for the preparation of optically pure isomers of pramipexole. The compound of formula G (X is a hydroxy group, the amino function is protected) can be resolved with chiral amines. The rearrangements, mentioned above, proceed with retention of the configuration at the migrating compound giving C in enantiomerically pure form.

WO 06/003471 discloses a process for the preparation of pramipexole comprising chiral chromatography, using a chiral mobile phase or preferably a chiral stationary phase.

Thus, the known processes for the preparation of (*S*)-pramipexole are not satisfactory, in particular for plant scale production, as they are lengthy, economically undesirable, the chemical and particularly optical purity of the products are not sufficient, the used starting materials are very expensive and they use hazardous chemical reagents.

In accordance with the foregoing an object of the present invention is to provide a new and improved process for the preparation of (*S*)-pramipexole which does not show the aforementioned drawbacks, in particular for plant scale production.

It has now been found that a process for the preparation of (*S*)-pramipexole, which employs novel intermediates, does not show the aforementioned drawbacks, in particular using this process (*S*)-pramipexole can be prepared in high chemical and optical purity and the requirements for the production in industrial scale are fulfilled.

It was surprisingly found that the process for the preparation of (*S*)-pramipexole is improved when intermediates having a hydroxy group in position 7 are used.

Therefore, the present invention relates to a process for the preparation of a compound of the formula I ((S)-pramipexole) 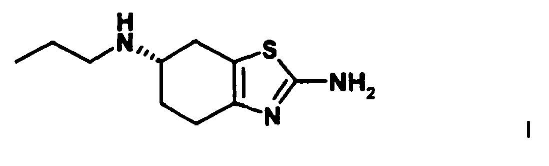 or a salt thereof, comprising the step of optically resolving a compound of the formula V 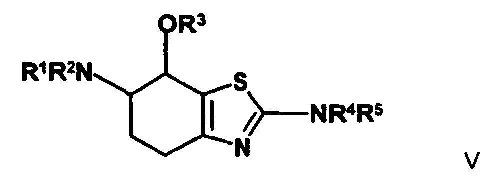 or a salt thereof, wherein R¹, R², R⁴ and R⁵ are independently H or an amino-protecting group, R³ is H or a hydroxy-protecting group and R¹ and R² and/or R⁴ and R⁵ may form a ring.

As protecting group for the optionally protected hydroxy group and the optionally protected amino groups usual protecting groups known to the person skilled in the art may be used. Suitable protecting groups are exemplified in WO 03/044011, the content of which is incorporated by reference herein.

The compound of formula V involves four stereoisomers: the racemic pairs of the *cis* and *trans* diastereoisomers of the 6 and 7 isomers. For the present application the term "optically resolving" defines a process step wherein the compound of the formula V is enriched in one of its 6-isomers, i.e. in its (6*S*) or (6*R*) isomer.

It was surprisingly found that when the racemic compounds of formula V are subjected to optical resolution e.g. with optical active, enantiomerically pure agents, preferably acids, in a suitable solvent the degree of enantiomeric enrichment in the resulting precipitate proves to be very high.

Great efficiency of preferential crystallization shows to be especially typical for compounds of the formula V, where the spatial relationship of the hydroxy and amino substituents is *cis*. In particular the compound of the formula V comprises > 50%, preferably> 75% and most preferably > 90% (such as 92.5%) *cis* diastereoisomers.

Suitable resolving agents, i.e. the optically active, enantiomerically pure agents, for the optical resolution of the compounds of formula V are enantiomerically pure acids, preferably dicarboxylic acids, including but not limited to D- or L-tartaric acid and their derivatives. The most preferred resolving agent is D-tartaric acid.

The choice of solvent can affect the salt-forming precipitation reaction and the subsequent separation of the resulting diastereomers by fractional crystallization. Suitable solvents include water, methanol, ethanol, 1-propanol, 2-propanol, acetone, dioxane, ethylacetate and mixtures thereof. The preferred solvent is water.

A suitable temperature of optical resolution is from 0°C to the boiling point of the solvent, preferably about 20°C.

In a preferred embodiment, the salts of the desired (6S,7S) and (6S,7R) isomers are less soluble than that of the (6R,7R) and (6R,7S) isomers and the (6S) isomers can be precipitated from the solution. The obtained salts preferably comprise at least 95% of (6S) isomers, more preferably at least 98% of (6S) isomers, and most preferred at least 99% of (6S) isomers. The degree of enrichment can be increased by further recrystallization of the desired enriched salts from a suitable solvent. Advantageously the same solvent is used which was applied for the resolution. The salts can be isolated in solvated or hydrated form.

In an example of the advantageous embodiment of the optical resolution process of the present invention, racemic compounds of the formula V containing cis and *trans* isomers in a ratio of 92.5:7.5 are reacted with D-tartaric acid in water as the solvent, whereby D-tartaric acid salts of the (6S) isomers separate out from the solution as a solid. The obtained salts can be neutralized by the addition of a base to set the corresponding optically enriched compounds of the formula VII free.

In a preferred embodiment of the present invention the compound of the formula V is obtained by reducing a compound of the formula IV 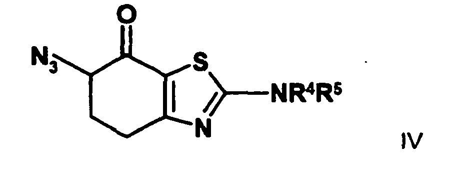 or a salt thereof, wherein R⁴ and R⁵ are defined as above. The reduction step can be carried out by usual reaction steps being well-known to the person skilled in the art, such as reaction with a complex metal hydride and catalytic hydrogenation. Both reactions can be carried out without isolation of the azido-alcohol intermediates.

Furthermore, the compound of the formula IV can be obtained by replacing the halogen substituent in a compound of the formula III 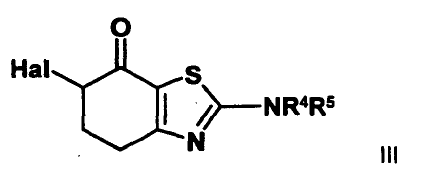 or a salt thereof, wherein Hal is a halogen atom and R⁴ and R⁵ are defined as above, by an azido group. This reaction can for example be carried out with sodium azide in a suitable solvent such as a mixture of water and dimethylformamide.

Furthermore, the compound of the formula III can for example be obtained by selectively monohalogenating a compound of the formula II 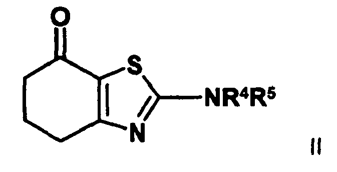 or a salt thereof, wherein R⁴ and R⁵ are defined as above.

Thus, in a preferred process of the present invention the compound of formula V can be prepared from 2-amino-7-oxo-4,5,6,7-tetrahydrobenzothiazole (compound of formula 11), the latter compound being in the prior art (e.g. Lehmann, G. et al. Z. Chem. 7, 422 (1967)). This compound of formula II can e.g. be selectively monobrominated in methanol to give the α-bromo-ketone as a compound of formula III. Substitution of the leaving group by azide performed with sodium azide in a mixture of water and dimethylformamide results in a compound of formula IV. The azido-ketone can be subjected to complex metal hydride reduction (e.g. NaBH₄) and subsequent catalytic hydrogenation, optionally using an appropriate catalyst, e.g. palladium on charcoal, whereby the hydroxy-amine, a compound of the formula V, can be obtained, without the isolation of the appropriate azido-alcohol intermediates.

In order to obtain (*S*)-pramipexole from the compound of the formula V it is further required to remove the optionally protected hydroxy group after optically resolving the compound of the formula V as described above. Thus, the present invention also relates to a process for the preparation of (S)-pramipexole or a salt thereof, comprising the step of removing the optionally protected hydroxy group from the compound of the formula IX 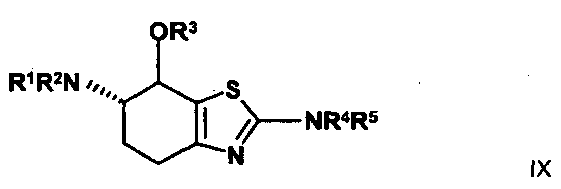 or a salt thereof, wherein R¹, R⁴ and R⁵ are independently H or an amino-protecting group, R² is H, an amino-protecting group or n-propyl, R³ is H or a hydroxy-protecting group and R¹ and R² and/or R⁴ and R⁵ may, if possible, form a ring.

The removal of the optionally protected hydroxy group can be achieved by a hydrogenolysis reaction, which is usually carried out in a strong acidic medium. The reaction is advantageously carried out in a suitable solvent such as formic acid, acetic acid or trifluoroacetic acid, optionally in the presence of a strong organic or inorganic acid such as methanesulfonic acid, benzenesulfonic acid, p-toluenesulfonic acid, hydrochloric acid, sulfuric acid or perchloric acid. The reaction is conveniently carried out at temperatures of between 0°C to 100°C, preferably at temperatures of between 15°C to 50°C. Care has to be taken in selection of the hydrogenation catalyst, as the sulfur present in the molecule can poison the catalyst. Palladium on carbon is the preferred catalyst. The hydrogenolysis proceeds at low- or medium-pressure, preferably at pressures of between 4 to 6 bar.

In a preferred embodiment of the step of removing the optionally protected hydroxy group in the process of the present invention R¹, R⁴ and R⁵ are H and R² is n-propyl in the compound of the formula IX. Furthermore, the step of converting a compound of the formula IX, wherein R² is H or an amino-protecting group, into a compound of the formula IX, wherein R² is n-propyl, may be carried out prior to removing the optionally protected hydroxy group.

Preferably the compound of the formula IX is selected from the following compounds of the formulae VI, VII and VIII: 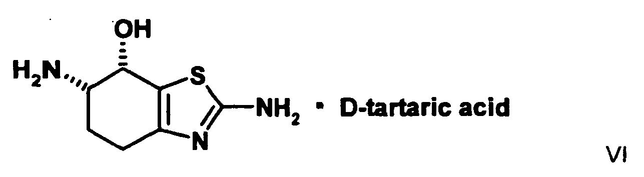 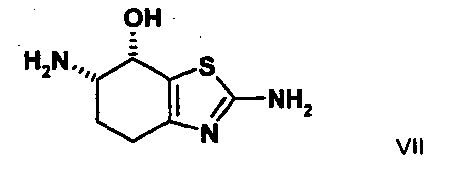 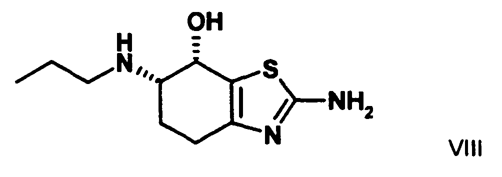 or salts thereof.

The compound of the formula IX can be obtained by optically resolving a compound of the formula V as described above.

In an alternative embodiment of the process for the preparation of a compound of the formula I of the present invention the compound of the formula IX is converted into a compound of the formula X 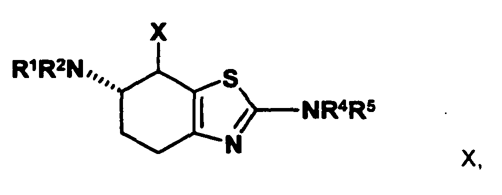 or a salt thereof, wherein R¹, R², R⁴ and R⁵ are defined as above for the compound of the formula IX and X is a leaving group, prior to conversion into a compound of formula I.

This alternative process involves the conversion of the compound of the formula IX into an analogous compound thereof by conversion of the optionally protected hydroxy group at position 7 into a leaving group. This leaving group is preferably a halogen, most preferably bromide. Alternatively alkylsulfonate or arylsulfonate esters like mesylate or tosylate groups can be used as leaving group X. The conversion of the hydroxy group into its bromoanalogue can be carried out in a solution of hydrogen bromide in acetic acid. Afterwards the bromide can be replaced by a hydride ion with the use of e.g. complex metal hydrates such as sodium triacetoxyborohydride, generated in situ from sodium borohydride.

The product of the hydrogenolysis reaction or the alternative process involving a compound of the formula X can be isolated as a free base (compound of the formula I) or as an acid addition salt and/or a solvate thereof, the preferred product is the dihydrochloride monohydrate (compound of the formula I x 2 HCl x 1 H₂O).

In a preferred embodiment of the process of the present invention, the compound of the formula IX, wherein R² is n-propyl, is obtained from a compound of formula IX, wherein R² is other than n-propyl.

According to EP 186 087 A1 the last step of the overall synthesis comprises reductive alkylation of 2,6-diamino-4,5,6,7-tetrahydrobenzothiazole (formula C, R₁ and R₂ are hydrogen) with a corresponding carbonyl compound (in the case of pramipexole: propionaldehyde) and a complex metal hydride such as sodium borohydride or sodium cyanoborohydride. The reaction is, however, low yielding, and the chemical purity of the obtained product does not meet the general requirements demanded from APIs. The composition of the reaction mixture is disadvantageous, containing starting material and overalkylated products in large quantities (from 10 to 30%).

It has now been surprisingly found that the presence of the optionally protected hydroxy group in position 7 results in a selective monopropylation of the amino substituent in position 6 of the compound IX. The reductive alkylation of the 7-hydroxy analogue to prior art compound C with the carbonyl compound, in particular propionaldehyde, and sodium borohydride shows to have good yield and a high chemical and optical purity. This reaction is preferably carried out in methanol and at temperatures of between -25°C and -20°C. The resulting compound can be isolated as free base or as a salt, preferably the sulfate salt.

The present invention further relates to a compound of the formula IV 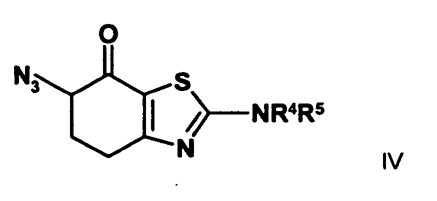 or a salt thereof, wherein R⁴ and R⁵ are independently H or an amino-protecting group and R⁴ and R⁵ may form a ring,
of the formula V 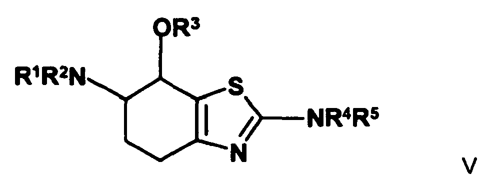 or a salt thereof, wherein R¹, R², R⁴ and R⁵ are independently H or an amino-protecting group, R³ is H or a hydroxy-protecting group and R¹ and R² and/or R⁴ and R⁵ may form a ring,
of the formula VI 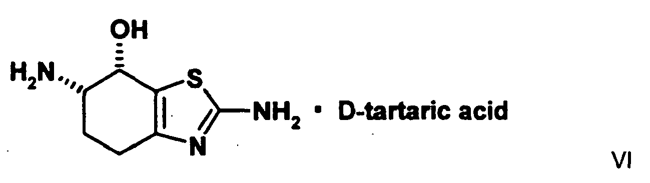 or a salt thereof,
of the formula VII 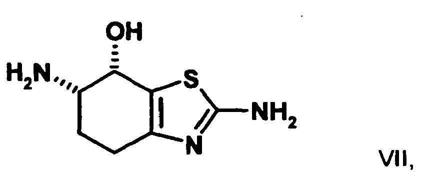 or a salt thereof,
of the formula VIII 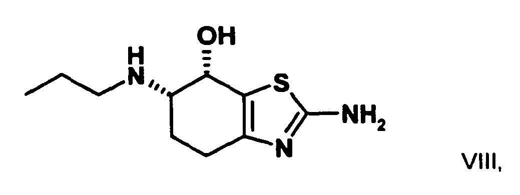 or a salt thereof,
of the formula IX 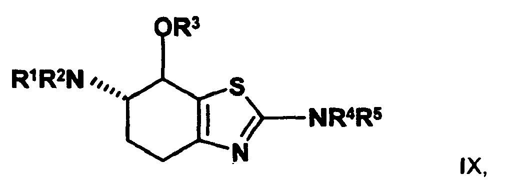 or a salt thereof, wherein R¹, R⁴ and R⁵ are independently H or an amino-protecting group, R² is H, an amino-protecting group or n-propyl, R³ is H or a hydroxy-protecting group and R¹ and R² and/or R⁴ and R⁵ may, if possible, form a ring,
and of the formula X 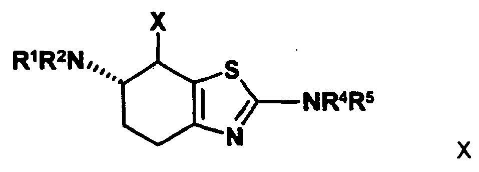 or a salt thereof, wherein R¹, R⁴ and R⁵ are independently H or an amino-protecting group, R² is H, an amino-protecting group or n-propyl, R¹ and R² and/or R⁴ and R⁵ may, if possible, form a ring, and X is a leaving group.

The processes of the present invention and the intermediates resulting therefrom are advantageous in the preparation of (S)-pramipexole, and in particular advantageous for the production in industrial scale. The resolution of the enantiomers takes place in a relatively early stage of the synthesis and is thereof efficient leading to a product with high chemical and optical purity. Thus, the present invention further relates to the use of the above intermediates for the preparation of (S)-pramipexole.

The present invention will now be further illustrated by the following examples which are not intended to be limiting.

### Example 1

### 2-Amino-6-bromo-7-oxo-4,5,6,7-tetrahydrobenzothiazole (compound of the formula III)

2-Amino-7-oxo-4,5,6,7-tetrahydrobenzothiazole hydrobromide (compound of the formula II) (610 g) (prepared by modification of the process known from Lehmann, G. et al. Z. Chem. 7, 422 (1967)) was suspended in methanol (4.160 ml). Concentrated sulfuric acid (302 g) was added to the stirred suspension at 18-23°C. The hydrobromide salt gradually solubilized during the addition until complete dissolution. Trimethyl orthoformate (390 g) was added to the solution in 90 min, the reaction mixture was stirred for a further 90 min at 20-23°C and then cooled to 0-2°C. Bromine (391 g) was dropwise added at 0-5°C within 2.5 to 3 h, the reaction mixture was stirred for a further 2 h at that temperature, and allowed to warm up slowly to room temperature during the night. The next day water (5.400 ml) was added to the suspension, and the so obtained solution is heated to 45-50°C. After 1 h at that temperature, it was cooled to 0-5°C and neutralized by adding saturated aqueous NaHCO₃ solution (8.800 ml). The precipitated product was collected by filtration, washed with water (2 x 1.000 ml) and air-dried to give 560-564 g of product. The yield 93%, m.p. 198-200°C.

### Example 2

### 2-Amino-6-azido-7-oxo-4,5,6,7-tetrahydrobenzothiazole (compound of the formula IV)

To a stirred suspension of 2-amino-6-bromo-7-oxo-4,5,6,7-tetrahydrobenzothiazole (compound of the formula III) (432.5 g) in a mixture of dimethylformamide (5.100 ml) and water (1.700 ml) was added sodium azide (135.9 g) in one portion at 5-7°C. The reaction mixture was allowed to warm up to room temperature in 5 h, an additional portion of sodium azide (34.8 g) was added and stirring was continued for 16 h. The product was precipitated from the solution by adding water (9.000 ml), filtered, washed with water (2 x 1.000 ml) and air-dried to give 309 g (84%) of (IV) with m.p. 172-176°C.

### Example 3

### 2,6-Diamino-7-hydroxy-4,5,6,7-tetrahydrobenzothiazole (compound of the formula V)

To a well-stirred suspension of 2-amino-6-azido-7-oxo-4,5,6,7-tetrahydrobenzothiazole (compound of the formula IV) (350 g) in methanol (3.700 ml), cooled to 5-8°C, was added sodium borohydride (55.4 g) in ten parts. Both the substrate and the reagent gradually solubilized during the addition until complete dissolution. Stirring was continued for 90 min, then the temperature was raised to 20-23°C. After changing the atmosphere to argon palladium on carbon (10 wt.%, 33.5 g) was added and the solution was subjected to hydrogenation at atmospheric pressure for 16-24 h. The catalyst was removed by filtration, the filtrate was concentrated to 2.000 ml, cooled to 0-5°C and acidified by the addition of 37% hydrochloric acid. Precipitated crystals were collected by suction and the cake was washed with cold methanol to give 450 g of crude dihydrochloric salt.

The solid product was dissolved in water (2.000 ml) and the solution was alkalized with 5 N aqueous NaOH solution. The mixture was stirred at 0-5°C for 90 min and precipitated crystals were filtered by suction and washed with cold water to give 233 g (75%) of the title product with m.p. 207-209°C. HPLC (purity, A%): 99,9% *(cis*/*trans:* 92.5:7.5).

### Example 4

### (6S,7S)-2,6-Diamino-7-hydroxy-4,5,6,7-tetrahydrobenzothiazole D-tartrate salt (compound of the formula VI)

A solution of D-tartaric acid (150.09 g) in water (500 ml) was added to the suspension of 2,6-diamino-7-hydroxy-4,5,6,7-tetrahydrobenzothiazole (compound of the formula V) (185.26 g) in water (2.000 ml). After complete dissolution white crystals precipitated, the mixture was stirred at 20°C for 2-3 h, the solid was filtered off, washed with water (2 x 300 ml) and methanol (1 x 300 ml). 159.91 g of product was obtained, m.p. 155-160°C, HPLC (purity, A%): 99.9% *(cis*/*trans:* 97.8:2.1), [α]_{D}²⁰: -59.6 (c = 1.0, H₂O).

To increase the optical purity of the D-tartrate salt, 126.8 g of the product was recrystallized from water, affording 116.1 g of salt. m.p. 155-160°C, HPLC (purity, A%): 100.0% *(cis*/*trans:* 99.6:0.4), [α]_{D}²⁰: -62.5 (c = 1.1, H₂O).

### Example 5

### (6S,7S)-2,6-Diamino-7-hydroxy-4,5,6,7-tetrahydrobenzothiazole (compound of the formula VII)

A chilled suspension of (6*S*,7*S*)-2,6-diamino-7-hydroxy-4,5,6,7-tetrahydrobenzothiazole D-tartrate salt (compound of the formula VI) (115.37 g) in 630 ml of water was alkalized by addition of 5 *N* NaOH solution. After complete dissolution off-white crystals precipitated, the mixture was stirred at 0-3°C for 90 min, the solid was filtered off and washed with water (2 x 75 ml). 49.88 g of the title product was obtained, m.p. 184-185°C, HPLC (purity, A%): 99.97% *(cis*/*trans:* 99.7:0.3).

### Example 6

### (6S,7S)-2-Amino-7-hydroxy-6-(n-propyl)amino-4,5,6,7-tetrahydrobenzothiazole sulfate salt (compound of the formula VIII)

To a solution of (6*S*,7*S*)-2,6-diamino-7-hydroxy-4,5,6,7-tetrahydrobenzothiazole (compound of the formula VII) (44.4 g) in methanol (720 ml) was added propionaldehyde (28.0 g) at -25 - -20°C. Stirring was continued for 3 h and NaBH₄ (6.3 g) was added in small portions during 50 min. After 2 h the reaction mixture was allowed to warm up slowly to 0°C and maintained at that temperature for 90 min. The reaction mixture was acidified by the addition of concentrated sulfuric acid. The precipitated crystals were filtered, washed with cold methanol (2 x 90 ml) to afford 76 g of crude product.

The solid was dissolved in water (355 ml) and the solution was alkalized with 5 N aqueous NaOH solution. The mixture was stirred at 0-5°C for 90 min and precipitated crystals were filtered by suction and washed with cold water to give 43.0 g of free base.

The product was dissolved in methanol (344 ml), cooled to 0-5°C, the solution was acidified by the addition of concentrated sulfuric acid, the precipitated crystals were filtered in 2 h, washed with cold methanol (2 x 60 ml) and air-dried to give 54.5 g (74%) of the title product. m.p. 152-153°C, HPLC (purity, A%): 99.1%, [α]_{D}²⁰: -67.1 (c = 1.0, H₂O).

### Example 7

### (S)-2-Amino-(n-propyl)amino-4,5,6,7-tetrahydrobenzothiazole, (S)-pramipexole free base (I)

A solution of (6*S*,7*S*)-2-amino-7-hydroxy-6-(*n*-propyl)amino-4,5,6,7-tetrahydrobenzothiazole sulfate salt (compound of the formula VIII) (26.3 g) in a mixture of formic acid (98-100%, 250 g) and concentrated sulfuric acid (22 g) containing palladium on carbon (10 wt.%, 8.8 g) was subjected to hydrogen pressure of 5.5 bar for 24 h. The reaction mixture was diluted with methanol (240 ml), the catalyst was removed by filtration, washed with methanol, the combined filtrate and washings were concentrated to 70-75 g. The syrupy residue was dissolved in water (260 ml), the solution was cooled to 10°C and alkalized with 5 N aqueous NaOH solution. The mixture was stirred at 10°C for 90 min and precipitated crystals were filtered by suction and washed with cold water to give 15.21 g (90%) of the title product with m.p. 126-128°C. HPLC (purity, A%): 99.1%.

### Example 8

### (S)-2-Amino-6-(n-propyl)amino-4,5,6,7-tetrahydrobenzothiazole dihydrochloride monohydrate, (S)-pramipexole dihydrochloride monohydrate (I•2 HCl•1 H₂O)

A solution of (S)-pramipexole free base (Ia) (24.30 g) in acetone (730 ml) was cooled to 0-3°C, its pH was adjusted to 1.5 by the addition of 37% hydrochloric acid. The mixture was stirred at that temperature for 2 h and the precipitated crystals were collected by filtration. The cake was washed with cold acetone and air-dried to give 34.16 g (98%) product with m.p. 278-280°C. HPLC (purity, A%): 99.95%, [α]_{D}²⁰: -67.3 (c = 1.0, MeOH), optical purity (A%) (HPLC, using a chiral, optically active stationary phase): 99.6% ee.

### Example 9

### (S)-Pramipexole dihydrochloride monohydrate (I•2 HCl•1 H₂O)

To a solution of (6*S*,7*S*)-2-amino-7-hydroxy-6-(*n*-propyl)amino-4,5,6,7-tetrahydrobenzothiazole (the free base, compound of the formula VIII) (2.27 g) in formic acid (98-100%, 31 g) was added concentrated hydrochloric acid (3.5 g). The so obtained solution of (6*S*,7*S*)-2-amino-7-hydroxy-6-(*n*-propyl)amino-4,5,6,7-tetrahydrobenzothiazole-2 HCl was subjected to hydrogenation on palladium on carbon (10 wt.%, 1.1 g) using the same reaction conditions as described in example 7. The reaction mixture was diluted with methanol (30 ml), the catalyst was removed by filtration, washed with methanol, the combined filtrate and washings were concentrated to 10 g. The syrupy residue was diluted with acetone (90 ml), the solution was cooled to 0-3°C, stirred at that temperature for 2 h and the precipitated crystals were collected by filtration. The cake was washed with cold acetone and air-dried to give 2.63 g (87%) product with m.p. 277-280°C.

## Claims

1. Process for the preparation of a compound of the formula I ((S)-pramipexole) 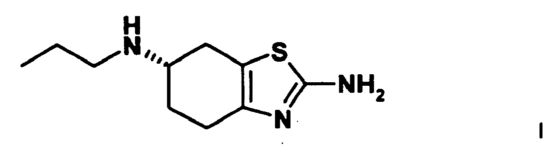 or a salt thereof, comprising the step of optically resolving a compound of the formula V 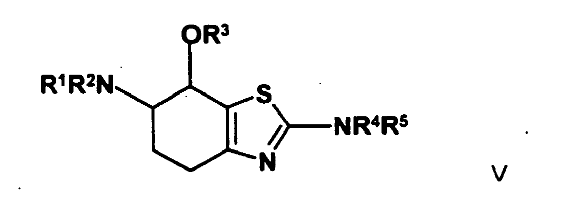 or a salt thereof, wherein R¹, R², R⁴ and R⁵ are independently H or an amino-protecting group, R³ is H or a hydroxy-protecting group and R¹ and R² and/or R⁴ and R⁵ may form a ring.

2. Process according to claim 1, wherein an enantiomerically pure acid is used to optically resolve the compound of the formula V.

3. Process according to claim 2, wherein the enantiomerically pure acid is a dicarboxylic acid, preferably D- or L-tartaric acid or a derivative thereof.

4. Process according to any of the preceding claims, wherein the compound of the formula V comprises > 50%, preferably > 75% and most preferably > 90% *cis* diastereoisomers.

5. Process according to any of the preceding claims, further comprising the step of reducing a compound of the formula IV 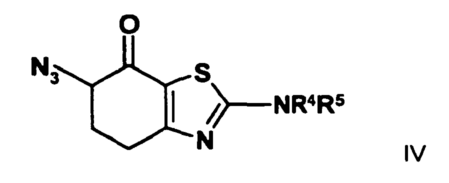 or a salt thereof, wherein R⁴ and R⁵ are defined as in claim 1, to obtain the compound of the formula V.

6. Process according to claim 5, further comprising the step of replacing the halogen substituent in a compound of the formula III 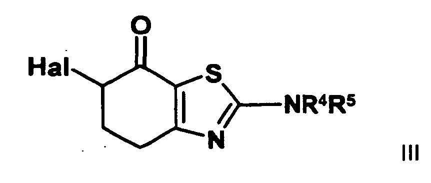 or a salt thereof, wherein Hal is a halogen atom and R⁴ and R⁵ are defined as in claim 1, by an azido group to obtain the compound of the formula IV.

7. Process according to claim 6, further comprising the step of halogenating a compound of the formula II 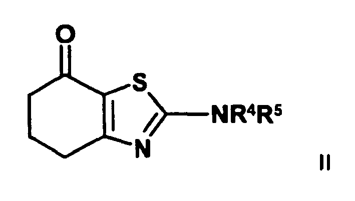 or a salt thereof, wherein R⁴ and R⁵ are defined as in claim 1, to obtain the compound of the formula III.

8. Process for the preparation of a compound of the formula I ((S)-pramipexole) 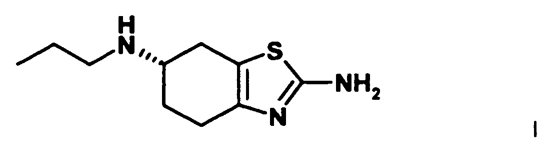 or a salt thereof, comprising the step of removing the optionally protected hydroxy group from a compound of the formula IX 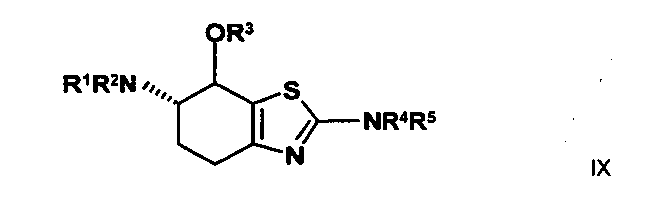 or a salt thereof, wherein R¹, R⁴ and R⁵ are independently H or an amino-protecting group, R² is H, an amino-protecting group or n-propyl, R³ is H or a hydroxy-protecting group and R¹ and R² and/or R⁴ and R⁵ may, if possible, form a ring.

9. Process according to claim 8, wherein R¹, R⁴ and R⁵ are H and R² is n-propyl.

10. Process according to claim 8, comprising the further step of converting a compound of the formula IX, wherein R² is H or an amino-protecting group, into a compound of the formula IX, wherein R² is n-propyl, prior to removing the optionally protected hydroxy group.

11. Process according to any of claims 8-10, wherein the compound of the formula IX is selected from the following compounds of the formulae VI, VII and VIII: 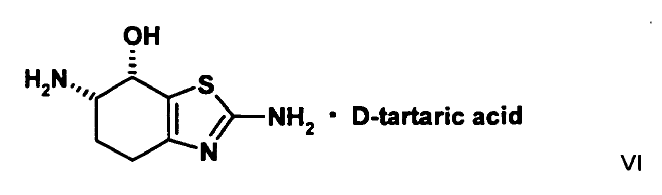 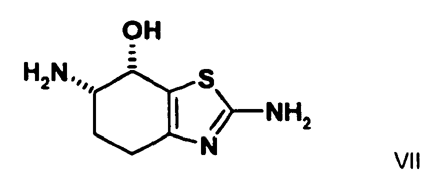 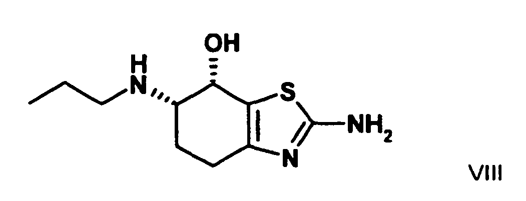 or salts thereof.

12. Process according to any of claims 8-11, wherein the compound of the formula IX is converted into a compound of the formula X 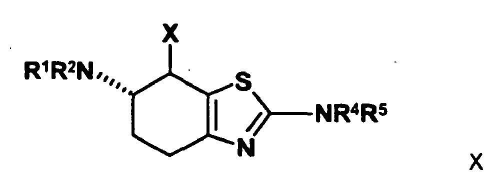 or 6 salt thereof, wherein R¹, R², R⁴ and R⁵ are defined as in claim 8 and X is a leaving group, prior to conversion into the compound of the formula I.

13. Process according to any of claims 8-12, wherein the compound of the formula IX is obtained by optically resolving a compound of the formula V 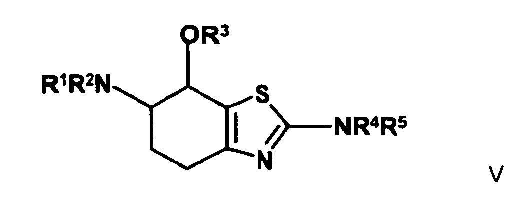 or a salt thereof, wherein R¹, R², R⁴ and R⁵ are independently H or an amino-protecting group, R³ is H or a hydroxy-protecting group and R¹ and R² and/or R⁴ and R⁵ may form a ring.

14. Compound of the formula IV 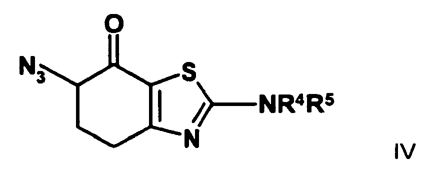 or a salt thereof, wherein R⁴ and R⁵ are independently H or an amino-protecting group and R⁴ and R⁵ may form a ring.

15. Compound of the formula V 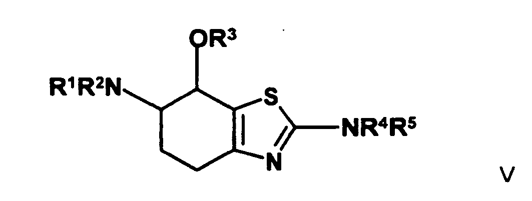 or a salt thereof, wherein R¹, R², R⁴ and R⁵ are independently H or an amino-protecting group, R³ is H or a hydroxy-protecting group and R¹ and R² and/or R⁴ and R⁵ may form a ring.

16. Compound of the formula VI 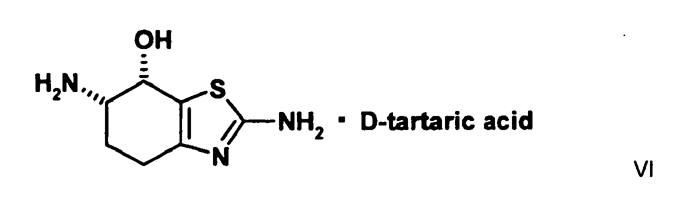 or a salt thereof.

17. Compound of the formula VII 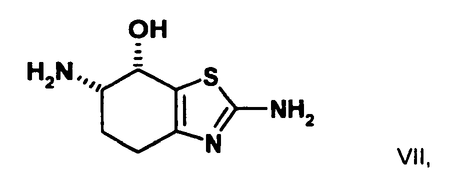 or a salt thereof.

18. Compound of the formula VIII 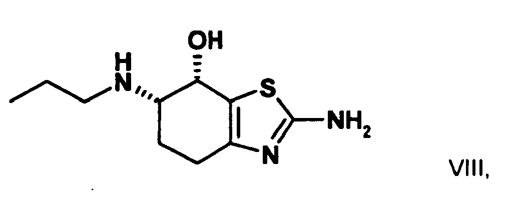 or a salt thereof.

19. Compound of the formula IX 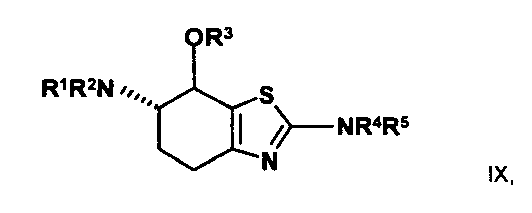 or a salt thereof, wherein R¹, R⁴ and R⁵ are independently H or an amino-protecting group, R² is H, an amino-protecting group or n-propyl, R³ is H or a hydroxy-protecting group and R¹ and R² and/or R⁴ and R⁵ may, if possible, form a ring.

20. Compound of the formula X 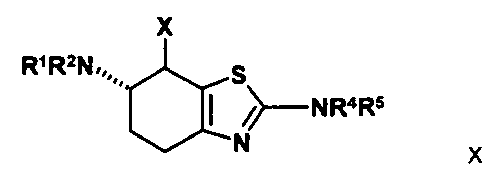 or a salt thereof, wherein R¹, R⁴ and R⁵ are independently H or an amino-protecting group, R² is H, an amino-protecting group or n-propyl, R¹ and R² and/or R⁴ and R⁵ may, if possible, form a ring, and X is a leaving group.

21. Use of any of the compounds according to claims 14-20 for the preparation of (*S*)-pramipexole.

## Patentansprüche

1. Verfahren zur Herstellung einer Verbindung der Formel I ((S)-Pramipexol) 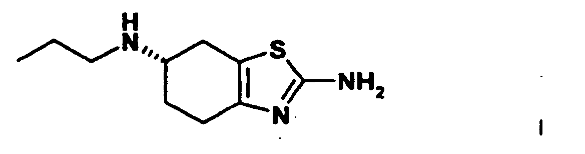 oder eines Salzes davon, umfassend den Schritt der optischen Trennung einer Verbindung der Formel V 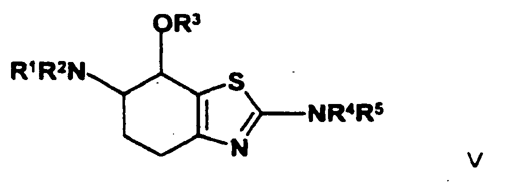 oder eines Salzes davon, worin R¹, R², R⁴ und R⁵ unabhängig voneinander H oder eine Amino-Schutzgruppe sind, R³ H oder eine Hydroxy-Schutzgruppe ist und R¹ und R² und/oder R⁴ und R⁵ einen Ring bilden können.

2. Verfahren nach Anspruch 1, wobei eine enantiomerenreine Säure zur optischen Trennung der Verbindung der Formel V verwendet wird.

3. Verfahren nach Anspruch 2, wobei die enantiomerenreine Säure eine Dicarbonsäure, vorzugsweise D- oder L-Weinsäure, oder ein Derivat davon ist.

4. Verfahren nach einem der vorhergehenden Ansprüche, wobei die Verbindung der Formel V > 50 %, vorzugsweise > 75 % und am stärksten bevorzugt > 90 % cis-Diastereoisomere umfaßt.

5. Verfahren nach einem der vorhergehenden Ansprüche, ferner umfassend den Schritt des Reduzierens einer Verbindung der Formel IV 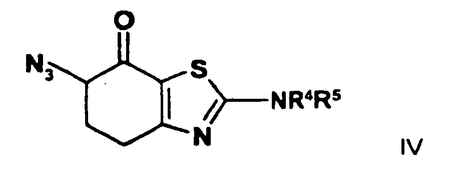 oder eines Salzes davon, worin R⁴ und R⁵ wie in Anspruch 1 definiert sind, unter Erhalt der Verbindung der Formel V.

6. Verfahren nach Anspruch 5, ferner umfassend den Schritt des Ersetzens des Halogensubstituenten in einer Verbindung der Formel III 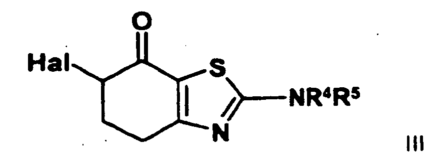 oder einem Salz davon, worin Hal ein Halogenatom ist und R⁴ und R⁵ wie in Anspruch 1 definiert sind, durch eine Azidogruppe unter Erhalt der Verbindung der Formel IV.

7. Verfahren nach Anspruch 6, ferner umfassend den Schritt der Halogenierung einer Verbindung der Formel II 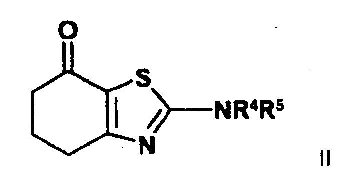 oder eines Salzes davon, worin R⁴ und R⁵ wie in Anspruch 1 definiert sind, unter Erhalt der Verbindung der Formel III.

8. Verfahren zur Herstellung einer Verbindung der Formel I ((S)-Pramipexol) 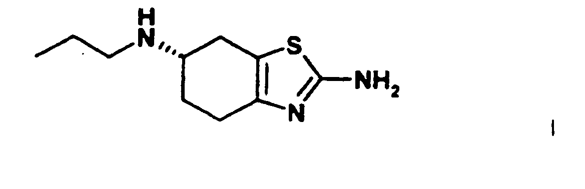 oder eines Salzes davon, umfassend den Schritt der Entfernung der gegebenenfalls geschützten Hydroxygruppe aus einer Verbindung der Formel IX 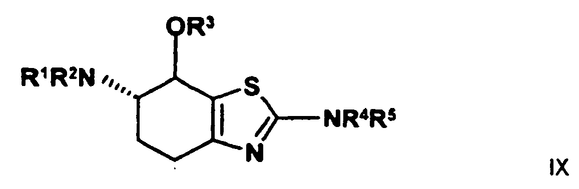 oder einem Salz davon, worin R¹, R⁴ und R⁵ unabhängig voneinander H oder eine Amino-Schutzgruppe sind, R² H, eine Amino-Schutzgruppe oder n-Propyl ist, R³ H oder eine Hydroxy-Schutzgruppe ist und R¹ und R² und/oder R⁴ und R⁵ gegebenenfalls einen Ring bilden können.

9. Verfahren nach Anspruch 8, worin R¹, R⁴ und R⁵ H sind und R² n-Propyl ist.

10. Verfahren nach Anspruch 8, umfassend den weiteren Schritt der Umwandlung einer Verbindung der Formel IX, worin R² H oder eine Amino-Schutzgruppe ist, in eine Verbindung der Formel IX, worin R² n-Propyl ist, bevor die gegebenenfalls geschützte Hydroxygruppe entfernt wird.

11. Verfahren nach einem der Ansprüche 8-10, wobei die Verbindung der Formel IX aus den folgenden Verbindungen der Formeln VI, VII und VIII: 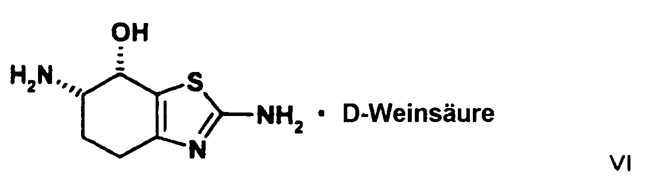 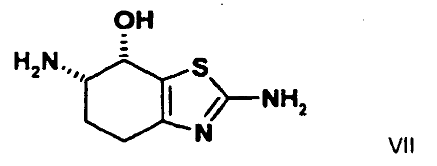 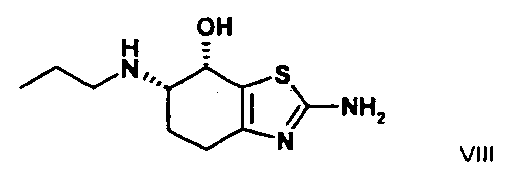 oder Salzen davon ausgewählt ist.

12. Verfahren nach einem der Ansprüche 8 - 11, wobei die Verbindung der Formel IX in eine Verbindung der Formel X 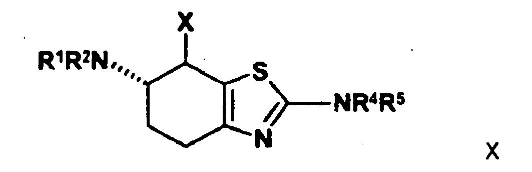 oder ein Salz davon, worin R¹, R², R⁴ und R⁵ wie in Anspruch 8 definiert sind und X eine Austrittsgruppe ist, vor der Umwandlung in die Verbindung der Formel I umgewandelt wird.

13. Verfahren nach einem der Ansprüche 8 - 12, wobei die Verbindung der Formel IX erhalten wird, indem eine Verbindung der Formel V 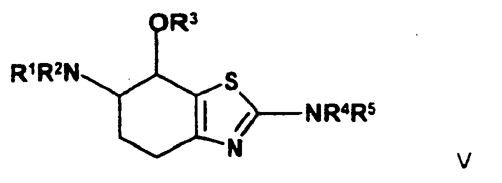 oder ein Salz davon, worin R¹, R², R⁴ und R⁵ unabhängig voneinander H oder eine Amino-Schutzgruppe sind, R³ H oder eine Hydroxy-Schutzgruppe ist und R¹ und R² und/oder R⁴ und R⁵ einen Ring bilden können, optisch getrennt wird.

14. Verbindung der Formel IV 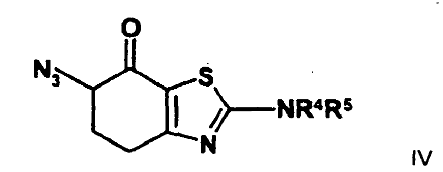 oder ein Salz davon, worin R⁴ und R⁵ unabhängig voneinander H oder eine Amino-Schutzgruppe sind und R⁴ und R⁵ einen Ring bilden können.

15. Verbindung der Formel V 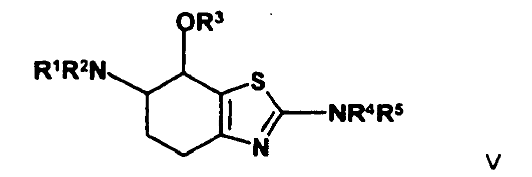 oder ein Salz davon, worin R¹, R², R⁴ und R⁵ unabhängig voneinander H oder eine Amino-Schutzgruppe sind, R³ H oder eine Hydroxy-Schutzgruppe ist und R¹ und R² und/oder R⁴ und R⁵ einen Ring bilden können.

16. Verbindung der Formel VI 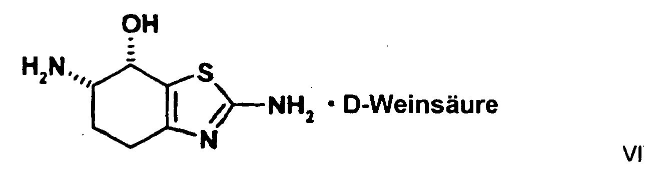 oder ein Salz davon.

17. Verbindung der Formel VII 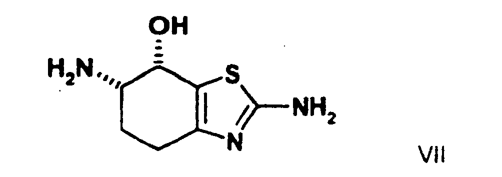 oder ein Salz davon.

18. Verbindung der Formel VIII 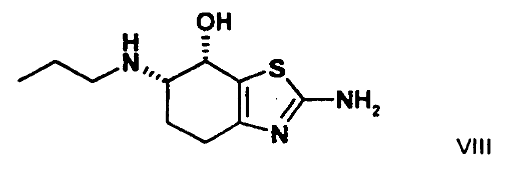 oder ein Salz davon.

19. Verbindung der Formel IX 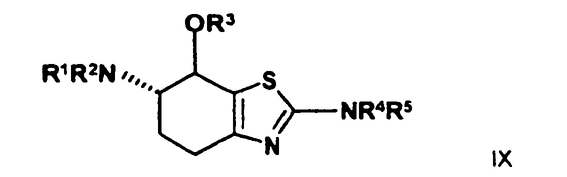 oder ein Salz davon, worin R¹, R⁴ und R⁵ unabhängig voneinander H oder eine Amino-Schutzgruppe sind, R² H, eine Amino-Schutzgruppe oder n-Propyl ist, R³ H oder eine Hydroxy-Schutzgruppe ist und R¹ und R² und/oder R⁴ und R⁵ gegebenenfalls einen Ring bilden können.

20. Verbindung der Formel X 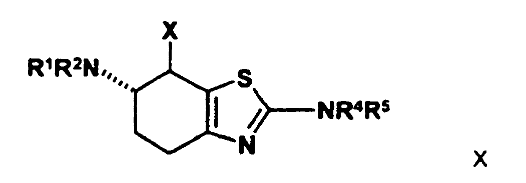 oder ein Salz davon, worin R¹, R⁴ und R⁵ unabhängig voneinander H oder eine Amino-Schutzgruppe sind, R² H, eine Amino-Schutzgruppe oder n-Propyl ist, R¹ und R² und/oder R⁴ und R⁵ gegebenenfalls einen Ring bilden können und X eine Austrittsgruppe ist.

21. Verwendung irgendeiner der Verbindungen nach den Ansprüchen 14-20 zur Herstellung von (S)-Pramipexol.

## Revendications

1. Procédé de préparation d'un composé de la formule I [(S)-pramipexole] 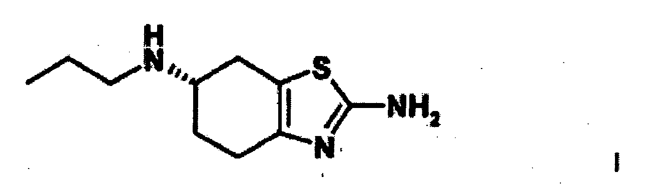 ou d'un sel de celui-ci, comprenant l'étape de résolution optique d'un composé de la formule V : 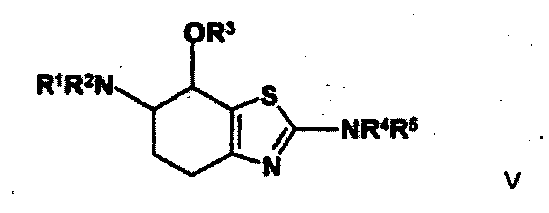 ou d'un sel de celui-ci, dans laquelle R¹, R², R⁴ et R⁵ représentent indépendamment H ou un groupe de protection d'amino, R³ est H ou un groupe de protection d'hydroxy et R¹ et R² et/ou R⁴ et R⁵ peuvent former un cycle.

2. Procédé suivant la revendication 1, dans lequel un acide énantiomériquement pur est utilisé pour résoudre optiquement le composé de la formule V.

3. Procédé suivant la revendication 2, dans lequel l'acide énantiomériquement pur est un acide dicarboxylique, avantageusement de l'acide D- ou L-tartrique ou un dérivé de celui-ci.

4. Procédé suivant l'une quelconque des revendications précédentes, dans lequel le composé de la formule V comprend > 50 %, avantageusement >75 % et le plus avantageusement >90 % de cis diastéréoisomères.

5. Procédé suivant l'une quelconque des revendications précédentes, comprenant de plus l'étape de réduction d'un composé de la formule IV : 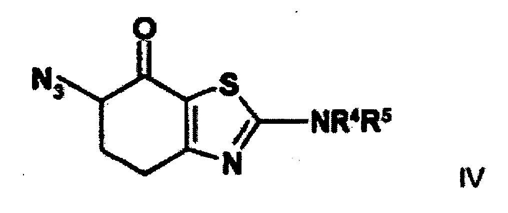 ou d'un sel de celui-ci, dans laquelle R⁴ et R⁵ sont tels que définis à la revendication 1, pour obtenir le composé de la formule V.

6. Procédé suivant la revendication 5, comprenant de plus l'étape de remplacement du substituant halogéné dans un composé de la formule III : 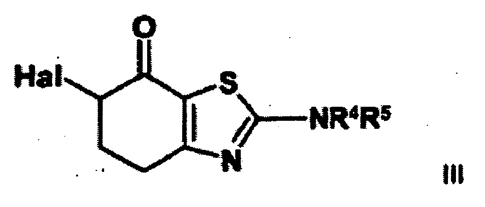 ou d'un sel de celui-ci, dans laquelle Hal est un atome d'halogène et R⁴ et R⁵ sont tels que définis à la revendication 1, par un groupe azido pour obtenir le composé de la formule IV.

7. Procédé suivant la revendication 6, comprenant de plus l'étape d'halogénation d'un composé de la formule II : 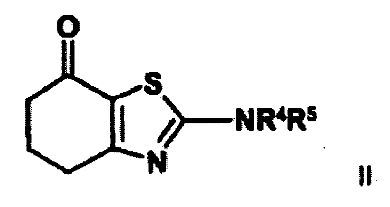 ou d'un sel de celui-ci, dans laquelle R⁴ et R⁵ sont tels que définis à la revendication 1, pour obtenir le composé de la formule III.

8. Procédé de préparation d'un composé de la formule I [(S)-pramipexole] 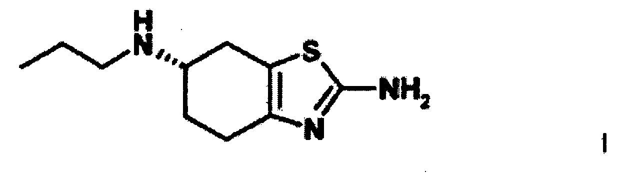 ou d'un sel de celui-ci, comprenant l'étape d'enlèvement du groupe hydroxy éventuellement protégé d'un composé de la formule IX: 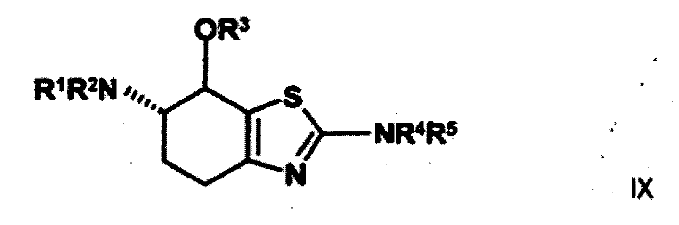 ou d'un sel de celui-ci, dans laquelle R¹, R⁴ et R⁵ représentent indépendamment H ou un groupe de protection d'amino, R² est H, un groupe de protection d'amino ou du n-propyle, R³ est H ou un groupe de protection d'hydroxy et R¹ et R² et/ou R⁴ et R⁵ peuvent, si possible, former un cycle.

9. Procédé suivant la revendication 8, dans lequel R¹, R⁴ et R⁵ représentent H et R² est du n-propyle.

10. Procédé suivant la revendication 8, comprenant l'étape ultérieure de conversion d'un composé de la formule IX, dans laquelle R² est H ou un groupe de protection d'amino, en un composé de la formule IX, dans laquelle R² est du n-propyle, avant l'enlèvement du groupe hydroxy éventuellement protégé.

11. Procédé suivant l'une quelconque des revendications 8 à 10, dans lequel le composé de la formule IX est choisi parmi les composés suivants des formules VI, VII et VIII : 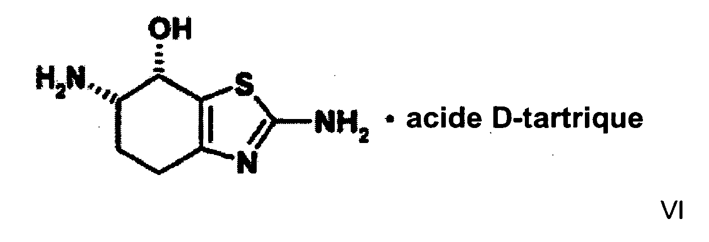 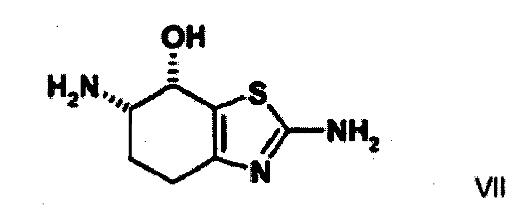 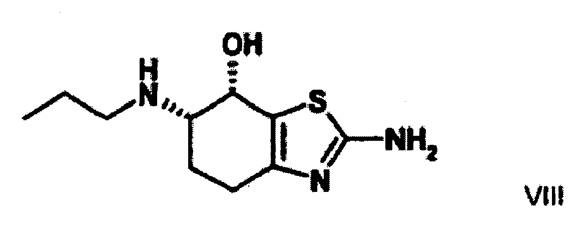 ou leurs sels.

12. Procédé suivant l'une quelconque des revendications 8 à 11, dans lequel le composé de la formule IX est converti en un composé de la formule X : 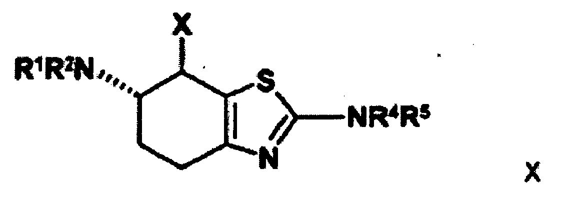 ou un sel de celui-ci, dans laquelle R¹, R², R⁴ et R⁵ sont tels que définis à la revendication 8 et X est un groupe partant, avant la conversion en le composé de la formule I.

13. Procédé suivant l'une quelconque des revendications 8 à 12, dans lequel le composé de la formule IX est obtenu par la résolution optique d'un composé de la formule V : 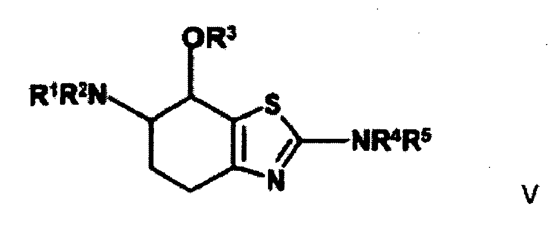 ou un sel de celui-ci, dans laquelle R¹, R², R⁴ et R⁵ représentent indépendamment H ou un groupe de protection d'amino, R³ est H ou un groupe de protection d'hydroxy et R¹ et R² et/ou R⁴ et R⁵ peuvent former un cycle.

14. Composé de la formule IV: 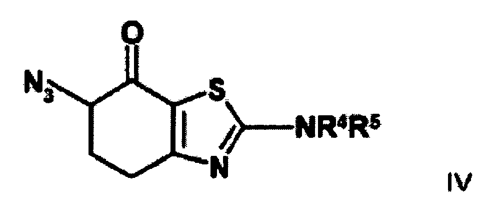 ou un sel de celui-ci, dans laquelle R⁴ et R⁵ représentent indépendamment H ou un groupe de protection d'amino et R⁴ et R⁵ peuvent former un cycle.

15. Composé de la formule V : 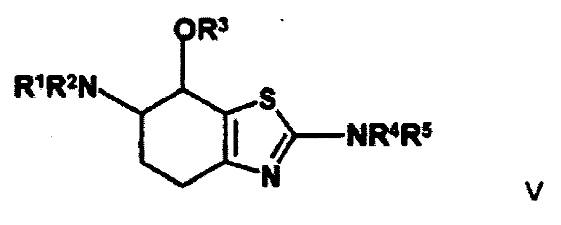 ou un sel de celui-ci, dans laquelle R¹, R², R⁴ et R⁵ représentent indépendamment H ou un groupe de protection d'amino, R³ est H ou un groupe de protection d'hydroxy et R¹ et R² et/ou R⁴ et R⁵ peuvent former un cycle.

16. Composé de la formule VI : 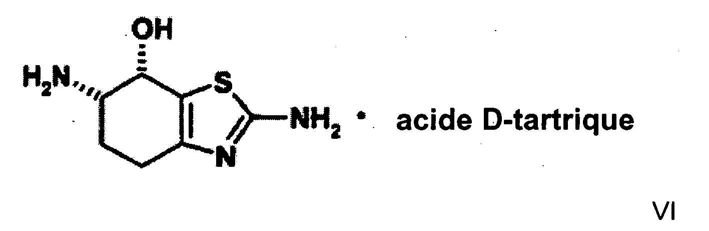 ou un sel de celui-ci.

17. Composé de la formule VII : 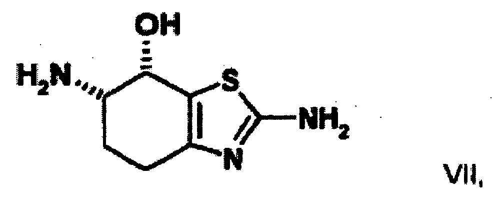 ou un sel de celui-ci.

18. Composé de la formule VIII : 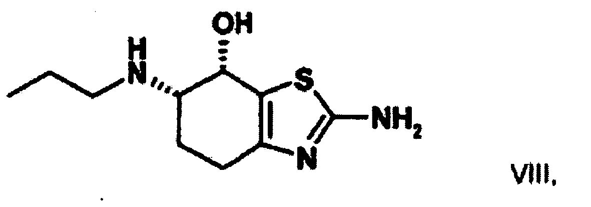 ou un sel de celui-ci.

19. Composé de la formule IX : 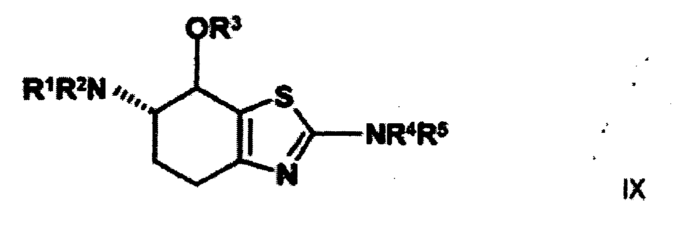 ou un sel de celui-ci, dans laquelle R¹, R⁴ et R⁵ représentent indépendamment H ou un groupe de protection d'amino, R² est H, un groupe de protection d'amino ou du n-propyle, R³ est H ou un groupe de protection d'hydroxy et R¹ et R² et/ou R⁴ et R⁵ peuvent, si possible, former un cycle.

20. Composé de la formule X : 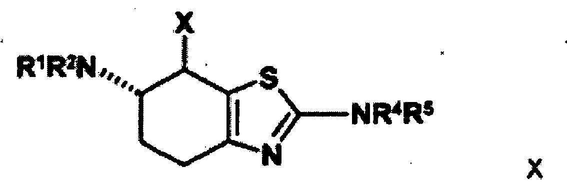 ou un sel de celui-ci, dans laquelle R¹, R⁴ et R⁵ représentent indépendamment H ou un groupe de protection d'amino, R² est H, un groupe de protection d'amino ou du n-propyle, R¹ et R² et/ou R⁴ et R⁵ peuvent, si possible, former un cycle, et X est un groupe partant.

21. Utilisation de l'un quelconque des composés suivant les revendications 14-20 pour la préparation de (S)-pramipexole.
